(19) Europäisches Patentamt
European Patent Office
Office européen des brevets

(11) Veröffentlichungsnummer: **0 122 533**
**B1**

(12) # EUROPÄISCHE PATENTSCHRIFT

(45) Veröffentlichungstag der Patentschrift: **25.01.89**

(21) Anmeldenummer: **84103642.9**

(22) Anmeldetag: **03.04.84**

(51) Int. Cl.⁴: **G 03 C 7/36** // C07C103/30, C07C103/38, C07C147/10, C07D233/90, C07D487/04, C07D235/14, C07D249/10

(54) **Farbfotografisches farbkupplerhaltiges Aufzeichnungsmaterial.**

(30) Priorität: **15.04.83 DE 3313721**

(43) Veröffentlichungstag der Anmeldung: **24.10.84 Patentblatt 84/43**

(45) Bekanntmachung des Hinweises auf die Patenterteilung: **25.01.89 Patentblatt 89/4**

(84) Benannte Vertragsstaaten: **DE FR GB IT**

(56) Entgegenhaltungen:
**DE-A-3 245 240**
**DE-A-3 409 886**
**FR-A-2 130 713**
**FR-A-2 169 879**
**GB-A-458 664**
**US-A-4 133 958**

**Die Akte enthält technische Angaben, die nach dem Eingang der Anmeldung eingereicht wurden und die nicht in dieser Patentschrift enthalten sind.**

(73) Patentinhaber: **Agfa- Gevaert AG, Patentabteilung, D-5090 Leverkusen 1 (DE)**

(72) Erfinder: **Renner, Günter, Dr., Wagnerstrasse 50, D-5060 Bergisch Gladbach 2 (DE)**
Erfinder: **Wolff, Erich, Dr., Balkhauser Weg 6, D-5650 Solingen (DE)**
Erfinder: **Sommer, Friedhelm, Dipl.- Chem., Walter-Flex- Strasse 19, D-5090 Leverkusen 1 (DE)**
Erfinder: **Stark, Herbert, Dr., Kölner Strasse 25, D-5800 Hagen 7 (DE)**
Erfinder: **Stieler, Dieter, Dipl.- Phys., Carl- Rumpff-Strasse 10, D-5090 Leverkusen 1 (DE)**
Erfinder: **Meier, Egon, Ing. grad., Karl- Friedrich-Gördeler- Strasse 30, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 122 533 B1

LIBER, STOCKHOLM 1989

**Beschreibung**

Die Erfindung betrifft ein farbfotografisches Aufzeichnungsmaterial mit mindestens einer Silberhalogenidemulsionsschicht und einem Gehalt an einem nicht diffundierenden einemulgierten α-Acylacetanilid-Gelbkuppler, dessen Anilidgruppe in 2-Stellung mit einem Halogenatom und mindestens in 4- und 5-Stellung mit je einer Alkoxygruppe substituiert ist.

Es ist bekannt, farbige fotografische Bilder durch chromogene Entwicklung herzustellen, d.h. dadurch, daß man bildmäßig belichtete Silberhalogenidemulsionsschichten in Gegenwart geeigneter Farbkuppler mittels geeigneter farbbildender Entwicklersubstanzen - sogenannter Farbentwickler - entwickelt, wobei das in Übereinstimmung mit dem Silberbild entstehende Oxidationsprodukt der Entwicklersubstanzen mit dem Farbkuppler unter Bildung eines Farbstoffbildes reagiert. Als Farbentwickler werden gewöhnlich aromatische, primäre Aminogruppen enthaltende Verbindungen, insbesondere solche vom p-Phenylendiamintyp verwendet.

An die Farbkuppler, sowie an die daraus durch chromogene Entwicklung erhaltenen Farbstoffe werden in der Praxis eine Reihe von Forderungen gestellt. So soll die Kupplungsgeschwindigkeit der Farbkuppler mit dem Oxidationsprodukt des Farbentwicklers möglichst groß sein und es soll eine möglichst hohe maximale Farbdichte erzielt werden können. Die Farbkuppler sowie die daraus erhaltenen Farbstoffe müssen hinreichend stabil sein gegenüber Licht, erhöhter Temperatur und Feuchtigkeit. Dies gilt sowohl für frisches Material, als auch für verarbeitetes Material. Beispielsweise darf der in den Bildweißen des verarbeiteten Materials noch vorhandene restliche Kuppler nicht vergilben. Außerdem sollten die Farbstoffe hinreichend beständig sein gegenüber gasförmigen reduzierenden oder oxidierenden Agentien. Sie müssen ferner diffusionsfest in der Bildschicht verankert sein und sollen sich bei der chromogenen Entwicklung als möglichst feines Korn abscheiden. Schließlich müssen die aus den Farbkupplern bei der chromogenen Entwicklung entstehenden Farbstoffe eine günstige Absorptionskurve aufweisen mit einem Maximum, das der Farbe des jeweils gewünschten Teilbildes entspricht, und möglichst geringen Nebenabsorptionen.

In besonderem Maße gelten die genannten Forderungen für Gelbkuppler, weil diese in farbfotografischen Aufzeichnungsmaterialien vielfach in der obersten farberzeugenden Schicht angeordnet sind und damit nicht nur besonders stark den Umwelteinflüssen ausgesetzt sind, sondern auch die darunterliegenden Schichten insbesondere hinsichtlich der Schärfe beeinflussen. Es sind daher alle Maßnahmen von Vorteil, durch die die Schichtbelastung besonders der gelbkupplerhaltigen Schicht reduziert werden kann.

Der Erfindung liegt die Aufgabe zugrunde für ein farbfotografisches Aufzeichnungsmaterial Gelbkuppler zur Verfügung zu stellen, die sich gut in verschiedenen Ölbildnern lösen und die, gelöst in einer möglichst geringen Menge Ölbildner, mit möglichst hoher Farbausbeute zu gelben Bildfarbstoffen mit einem Absorptionsmaximum unterhalb von 450 nm kuppeln. Kuppler wie auch Farbstoffe sollen stabil gegen den Einfluß von Wärme, Feuchtigkeit und Licht sein. Die Kuppler sollen im nahen UV-Bereich ein gutes Absorptionsvermögen haben und durch Änderung des pH-Wertes praktisch nicht in ihrer Kupplungsaktivität beeinflußt werden.

Gegenstand der Erfindung ist ein farbfotografisches Aufzeichnungsmaterial mit mindestens einer lichtempfindlichen Silberhalogenidemulsionsschicht und einem dieser zugeordneten nicht diffundierenden Gelbkuppler, dadurch gekennzeichnet, daß der Gelbkuppler der folgenden Formel entspricht

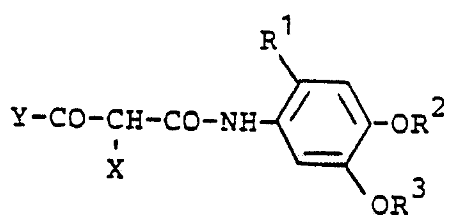

worin bedeuten:

Y eine p-Alkoxyphenylgruppe wie im Patentanspruch definiert;
X ein Wasserstoffatom oder eine bei Farbkupplung abspaltbare Gruppe;
$R^1$ ein Halogenatom, z. B. Fluor, Chlor oder Brom;
$R^2$ und $R^3$ gleiche oder verschiedene Alkylreste mit je 1 bis 20 C-Atomen, einschließlich substituierter Alkylreste, z. B. Benzyl.

Die erfindungsgemäßen Gelbkuppler sind darüber hinaus mit Ballastresten ausgestattet, die es ermöglichen, die Gelbkuppler in den üblicherweise bei fotografischen Aufzeichnungsmaterialien verwendeten hydrophilen Kolloiden diffusionsfest einzulagern. Hierzu sind vorzugsweise organische Reste geeignet, die im allgemeinen geradkettige oder verzweigte aliphatische Gruppen und gegebenenfalls auch carbocyclische oder heterocyclische aromatische Gruppen mit im allgemeinen 8 bis 20 C-Atomen enthalten. Mit dem übrigen Molekülteil sind diese Reste entweder direkt oder indirekt, z. B. über eine der folgenden Gruppen verbunden: -NHCO-, $-NHSO_2-$, -NR-, wobei R Wasserstoff oder Alkyl bedeutet, -O- oder -S-. Da die Diffusionseigenschaften von der Molekülgröße der verwendeten Gesamtverbindung abhängen, genügt es in bestimmten Fällen, z. B. wenn das verwendete Gesamtmolekül groß genug ist, als Ballastreste auch

kürzerkettige Reste zu verwenden.

Eine durch X dargestellte bei Farbkupplung abspaltbare Gruppe ist bevorzugt eine über ein Sauerstoffatom oder über ein Stickstoffatom, insbesondere ein Ringstickstoffatom angeknüpfte cyclische Gruppe, z. B. ein über ein Ringstickstoffatom angeknüpfter, gegebenenfalls substituierter 5- oder 6-gliedriger heterocyclischer Ring. Solche auch als Fluchtgruppen bezeichnete abspaltbare Gruppen verleihen üblicherweise dem Kuppler das Verhalten eines 2-Äquivalentkupplers, d.h. der Kuppler benötigt zur Farbkupplung nur halbsoviel entwickelbares Silberhalogenid, wie der entsprechende 4-Äquivalentkuppler, bei dem X ein Wasserstoffatom bedeutet. Beispiele für geeignete Fluchtgruppen sind im folgenden angegeben.

**Fluchtgruppen**

1) 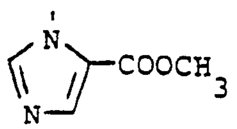

2) 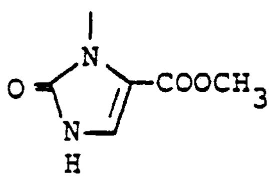

3) 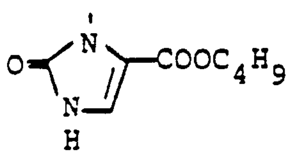

4) 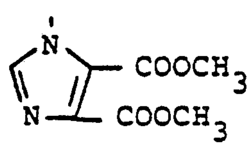

5) 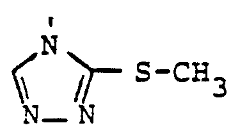

6) 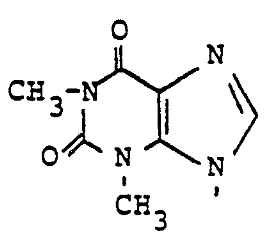

**Fluchtgruppen** (fortsetzung)

7) 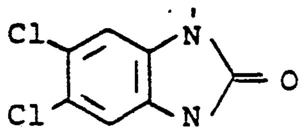

8) 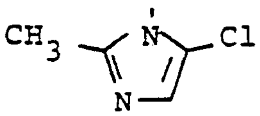

9) 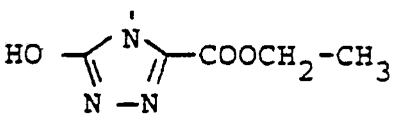

10) 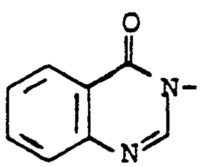

11) 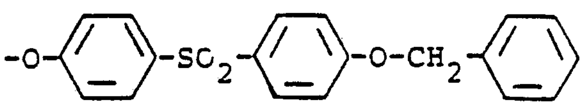

12) 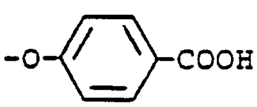

13) 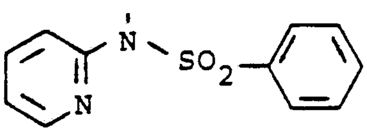

**Fluchtgruppen** (fortsetzung)

14) 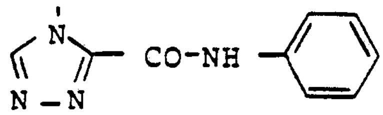

15) 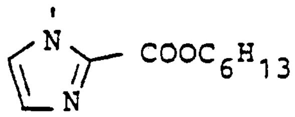

16) 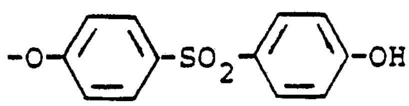

17) 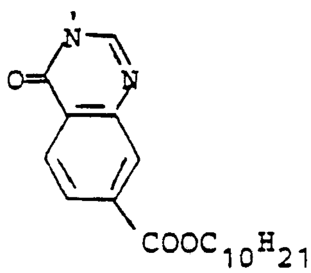

18) 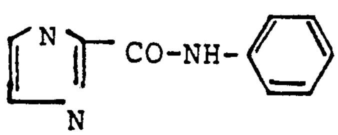

Beispiele erfindungsgemäßer Gelbkuppler sind im folgenden aufgeführt. Die in der Spalte X angegebene Zahl bezeichnet die entsprechende in der vorausgegangenen Auflistung angegebene Fluchtgruppe.

| Kuppler | Y | X | $R^1$ | $R^2$, $R^3$ |
|---|---|---|---|---|
| 1 | 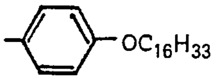 | 11 | Cl | $-CH_3$ |
| 2 | " | 9 | Cl | $-CH_3$ |
| 3 | " | 7 | Cl | $-CH_3$ |
| 4 | " | 2 | Cl | $-CH_3$ |
| 5 | " | 6 | Cl | $-CH_3$ |
| 6 | " | 18 | Cl | $-CH_3$ |
| 7 | " | 3 | Cl | $-CH_3$ |
| 8 | " | 5 | Cl | $-CH_3$ |
| 9 | " | 12 | Cl | $-CH_3$ |
| 10 | " | 13 | Cl | $-CH_3$ |
| 11 | " | 10 | Cl | $-CH_3$ |
| 12 | " | 5 | Br | $-CH_3$ |
| 13 | " | 4 | Br | $-CH_3$ |
| 14 | " | 1 | Br | $-CH_3$ |
| 15 | " | 1 | F | $-CH_3$ |
| 16 | " | 6 | Cl | $-CH(CH_3)_2$ |
| 17 | " | 2 | Cl | $-CH(CH_3)_2$ |
| 18 | " | 11 | Cl | $-CH(CH_3)_2$ |
| 19 | " | 11 | Br | $-CH(CH_3)_2$ |
| 20 | " | 10 | Br | $-CH(CH_3)_2$ |
| 21 | " | 14 | Cl | $-CH(CH_3)_2$ |
| 27 | 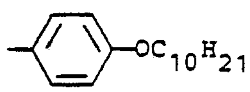 | 7 | Cl | 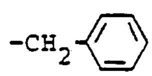 |
| 28 | " | 11 | Cl | " |
| 29 | " | 1 | Cl | " |
| 30 | " | 2 | Br | " |
| 31 | 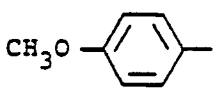 | 6 | Cl | $-C_8H_{17}$ |
| 32 | " | 15 | Cl | $-C_8H_{17}$ |
| 33 | " | 2 | Cl | $-C_8H_{17}$ |
| 34 | " | 11 | Cl | $-C_8H_{17}$ |
| 35 | " | 2 | Cl | $-C_8H_{17}$ |
| 40 | 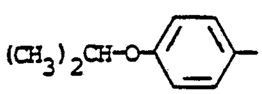 | 2 | Cl | $-C_6H_{13}$ |
| 41 | " | 7 | Cl | $-C_6H_{13}$ |
| 42 | " | 9 | Cl | $-C_6H_{13}$ |

**Kuppler 59**

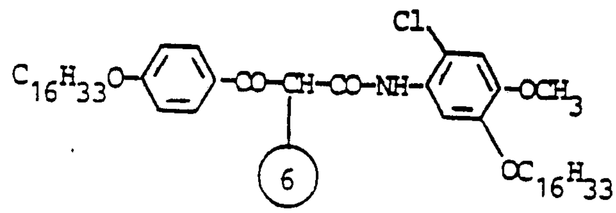

**Kuppler 60**

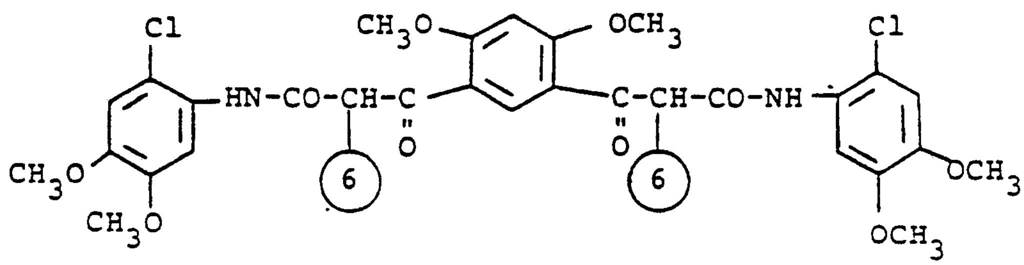

Die Synthese der erfindungsgemäßen Gelbkuppler erfolgt nach literaturbekannten Methoden: Durch Alkylierung von Brenzcatechin, Halogenierung des erhaltenen Diethers, anschließende Nitrierung und Reduktion des mehrfach substituierten Nitrobenzols wird das substituierte Anilin erhalten, das unter üblichen Reaktionsbedingungen mit dem ß-Ketoester kondensiert wird. Durch geeignete Oxidation des 4-Äquivalentkupplers wird in ebenfalls bekannten Reaktionsschritten die bei der Farbkupplung abspaltbare Gruppe (Fluchtgruppe) in das Molekül eingeführt. Die Synthese der erfindungsgemäßen Verbindungen sei an den nachfolgenden (präparativen) Beispielen gezeigt:

**Kuppler 1**

1.1 4-Chlorbrenzkatechindimethylether

34,5 g (0,25 mol) Brenzkatechindimethylether werden in 50 ml Eisessig gelöst. Dazu gibt man innerhalb 90 min bei 0°C eine Lösung von 33,8 g (0,25 mol) Sulfurylchlorid. Man rührt 30 min nach und fällt in Wasser aus. Ausbeute 37 g = 85,7 %.

1.2 4-Chlor-5-nitro-1,2-dimethoxybenzol

Zu 220 ml 65 %-ige Salpetersäure und 300 ml Wasser werden bei einer Temperatur von maximal 70°C 349,5 g Verbindung 1.1 langsam zugetropft. Man rührt 2 h bei 65°C nach, kühlt ab und saugt das ausgefallene Reaktionsprodukt ab. Ausbeute 392 g = 90 % d. Th. hellgelbe Kristalle; Fp. 106 - 108°C.

1.3 2-Chlor-4,5-Dimethoxyanilin

100 g des Produktes 1.2 werden in 1000 ml Ethanol bei 10 bar Wasserstoffdruck und 50°C mit Raney-Nickel als Katalysator innerhalb 4 h hydriert. Man saugt von Raney-Nickel ab und fällt das Amin mit wäßriger Salzsäure aus. Das HCl-Salz wurd abgesaugt, wiederum in Wasser verrührt und mit NaOH neutralisiert. Das nun ausgefallene freie Amin wird abgesaugt.
Ausbeute 66 g = 77 % d.Th.; hellgrau gefärbtes Pulver

1.4 α-(4-Cetyloxybenzoyl)-2-chlor-4,5-dimethoxy-acetanilid

375,4 g (2,0 mol) des Produktes 1.3, 920 g (2,2 mol) p-Cetyloxybenzoylessigester und 1300 ml Xylol werden zum Sieden erhitzt. Man destilliert 1100 ml Xylol ab und rührt die Lösung in 5000 ml Butanol aus. Nach dem Abkühlen wird abgesaugt. Der Filterrückstand wird mehrfach mit kaltem Methanol gewaschen.
Ausbeute: 920 g = 80 % d. Th. weiße Kristalle Fp. 120°C.

1.5 α-chlor- α-(4-cetyloxybenzoyl)-2'-chlor-4',5'-dimethoxyacetanilid

574 g (1,0 mol) des Produktes 1.4 werden in 1700 ml Methylenchlorid gelöst und mit 140 g (1,04 mol) Sulfurylchlorid bei Raumtemperatur chloriert. Das Methylenchlorid wird im Vakuum abdestilliert; der verbleibende Rest wird ohne Reinigung zur folgenden Reaktion eingesetzt.

1.6 α-[4-(4'-Benzyloxybenzoylsulfonyl)-phenoxy]-(4''-cetyloxybenzoyl)-2'''-chlor-4''',5'''-dimethoxyacetanilid

25 g (0,041 mol) Produkt 1.5 wurden in 125 ml Dimethylformamid mit 16,8 g (0,049 mol) 4-(4'-Benzyloxyphenylsulfonyl)-phenol gelöst. Bei 50 - 60°C werden 5,4 g Natriummethylat, gelöst in 18 ml Methanol, zugetropft. Man rührt noch 30 min bei 50°C und rührt danach in Eiswasser und 50 ml konzentrierte Salzsäure aus. Das Produkt wird abgesaugt, gewaschen und getrocknet. Rohausbeute: 38 g Man kristallisiert aus Acetonitril um und erhält 25 g = 67 % d. Th. weiße Kristalle vom Schmelzpunkt 112-114°C.

**Kuppler 2**

25 g des Produktes 1.4 werden wie unter 1.5 beschrieben chloriert. Der Rückstand wird in 300 ml Acetonitril gelöst und zu einer Mischung von 7,1 g 3-Carbethoxy-5-hydroxy-1,2,4-triazol und 20 ml Diazabicycloundecen bei 50°C getropft. Man rührt 1 h und fällt in saure wäßrige Lösung aus. Die organische Phase wird abgetrennt und eingedampft. Der Rückstand wird aus Ethanol umkristallisiert. Man erhält 26,5 g = 62 % d. Th. des Kupplers 2. Weiße Kristalle Fp. 107 - 139°C.

**Kuppler 3**

22 g des Produktes 1.5 und 11 g 5,6-Dichlor-2-ethylbenzimidazolon werden in 180 ml Dimethylacetamid gelöst. Dazu werden bei 50°C 15 ml Tetramethylguanidin getropft. Man rührt noch 1 h in 1800 ml 5°C kalte 1n-Salzsäure aus und nimmt das ausgefallene Öl mit Essigester auf. Die organische Phase wird gewaschen, getrocknet und eingeengt. Der Rückstand wird säulenchromatographisch mit einer Mischung von Cyclohexan und Essigester als Elutionsmittel gereinigt. Ausbeute 15 g = 52 % d.Th. Fp. 109 - 110°C.

In gleicher Weise wie bei Kuppler 1 beschrieben werden die Kuppler 4 bis 11 synthetisiert.

**Kuppler 12**

12.1 4-Brombrenzcatechindimethylether

69 g (0,5 mol) Dimethoxybenzol werden in 200 ml Methylenchlorid gelöst. Bei 0°C tropft man 80 g (0,5 mol) Brom dazu. Man rührt noch 1 h bei gleicher Temperatur nach, wäscht die organische Phase mit Wasser und destilliert das Lösungsmittel ab. Ausbeute nach GC 89,5 %.

Die Verbindung 12.1 wird in analoger Weise wie unter 1.2 bis 1.5 beschrieben umgesetzt.

12.6 19,6 g (0,03 mol) der Verbindung 12.5 werden in 150 ml Hexamethylphosphorsäuretriamid gelöst. Dazu werden 4,6 g (0,04 mol) 3-Methylthio-1,2,4-triazol gegeben. Zur erhaltenen Lösung tropft man innerhalb 2 h bei 30°C 10 ml Triethylamin. Man rührt noch 30 min nach und fällt in 1000 ml 1n-Salzsäure. Der Rückstand wird in Ethylacetat aufgenommen mit Wasser mehrfach gewaschen, getrocknet und eingedampft. Umkristallisation aus Ethanol ergibt weiße Kristalle vom Fp. 85 - 89°C. Ausbeute 12,2 g ≙ 61 % d. Th.

Die Vorstufe 15.1 wurde durch Diazotierung von 4-Aminobrenzcatechindimethylether in Gegenwart von Borfluorwasserstoffsäure und Zersetzen des Diazoniumtetrafluoroborates mit Kupfer-I-chlorid bei 60 - 80°C erhalten.

Der Kuppler 15 wurde durch Reaktionen analog zu dem vorgenannten erhalten.

Für die Kuppler 16 bis 21 wurde 1,2-Diisopropyloxybenzol als Ausgangsverbindung eingesetzt.

Die Kuppler 22 bis 26 wurden unter Verwendung von o-Cetyloxybenzoylessigester erhalten.

Die höheren Anilinoether (vgl. Kuppler 31 bis 44 und 54 bis 57) wurden durch Alkylierung von Brenzcatechin mit den entsprechenden Alkylbromiden in guter Ausbeute erhalten.

Für Kuppler 33 erwies sich folgendes Syntheseverfahren vorteilhaft:

29 g (0,05 mol) des 4-Äquivalentkupplers und 7,6 g Imidazolcarbonsäuremethylester werden in 150 ml Aceton gelöst. Dazu wurde bei Raumtemperatur langsam eine Lösung von 10 g Brom in 50 ml Acetonitril getropft. Es wurde mit Wasser ausgefällt, aufgenommen in Ethanol, gewaschen, abgetrennt, getrocknet und eingedampft. Der Rückstand konnte aus Ethanol umkristallisiert werden.

Ausbeute 23 g = 66 % d. Th. einer weißen kristallinen Substanz vom Fp. 125 - 127°C.

Kuppler 59 wurde durch Chlorierung von 2-Methoxyphenol, Alkylierung der erhaltenen Verbindung mit Cetylbromid und im übrigen analog zu den bereits beschriebenen Reaktionsschritten erhalten.

Die erfindungsgemäßen Gelbkuppler mit dem 2-Halogen-4,5-dialkoxyanilid-Rest weisen offenbar aufgrund des Vorhandenseins der benachbarten Alkoxygruppen - der Anilidrest kann zusätzlich weitere Alkoxygruppen enthalten - genügend hydrophile Eigenschaften auf, um auch ohne besondere Entwicklerzusätze, wie beispielsweise Benzylalkohol, die als Vermittler zwischen hydrophiler und hydrophober Phase anzusehen sind, die Entwicklung der Farbstoffe aus Farbkuppler mit der geforderten Empfindlichkeit und maximalen Farbdichte zu ermöglichen. Diese Wirkung der hydrophilen Zentren (Etherbindungen) hat auch einen positiven Einfluß auf das in DE-A-2 456 076 beschriebene "konkurrierende Reaktionsverhältnis" von Farbkupplern.

Der Ausdruck "konkurrierendes Reaktionsverhältnis" bezieht sich auf das Verhältnis der relativen Reaktionsfähigkeiten eines Gelbkupplers, einerseits in Gegenwart und andererseits in Abwesenheit eines konkurrierenden Kupplers wie Zitrazinsäure, H-Säure oder Weißkuppler gemäß der folgenden Gleichung

$$KRV = \frac{D_0}{D_1}$$

$D_0$ = maximale Farbdichte einer fotografischen Schicht, die in einem Farbentwickler ohne konkurrierenden Kuppler entwickelt wurde

$D_1$ = maximale Farbdichte der gleichen Schicht, die in einem Farbentwickler mit konkurrierendem Kuppler entwickelt wurde.

Der oben beschriebene positive Einfluß der erfindungsgemäßen Gelbkuppler in fotografischen Schichten äußert sich in einem kleineren konkurrierenden Reaktionsverhältnis. Die erfindungsgemäßen neuen Gelbkuppler haben gegenüber bekannten Gelbkupplern den Vorteil, daß sie bei der Farbkupplung durch die Anwesenheit von Konkurrenzkupplern weniger beeinflußt werden. Dies zeigt sich in einem kleineren konkurrierenden Reaktionsverhältnis bzw. in einer höheren Farbausbeute bei entsprechender Entwicklung. Die Vorteile einer höheren Farbausbeute, wie beispielsweise geringerer Kuppler- und Silberauftrag, speziell in der Gelbschicht, sind bekannt und bedürfen hier keiner näheren Erläuterung.

Die erfindungsgemäßen Gelbkuppler zeichnen sich weiterhin vor allem durch eine ausgezeichnete Löslichkeit und geringe Kristallisationstendenz in organischen Lösungsmitteln, insbesondere in mit Wasser nicht mischbaren Lösungsmitteln mit hohem Siedepunkt, wie z. B. Trikresylphosphat-Isomerengemisch oder Dibutylphthalat aus. Auch dies wirkt sich günstig im Hinblick auf eine geringere Schichtbelastung aus.

Außerdem besitzen sie eine hervorragende Diffusionsfestigkeit in fotografischen Schichten, sowohl beim Gießvorgang als auch während der fotografischen Verarbeitung.

Ein weiterer Vorteil der erfindungsgemäßen Gelbkuppler ist ihre hohe Stabilität gegenüber Feuchtigkeit und Wärme, sowie auch die Stabilität der aus ihnen hergestellten gelben Farbstoffe gegenüber Wärme, Feuchtigkeit und Lichteinstrahlung.

Die Gelbkuppler liefern gelbe Farbstoffe mit einem erwünschten Adsorptionsmaximum unterhalb 450 nm. Sie weisen selbst ein hohes Absorptionsvermögen im nahen UV-Bereich auf, was sich günstig auf die Stabilität der in den tieferliegenden Schichten erzeugten Bildfarbstoffe auswirkt.

Schließlich sind die Gelbkuppler der vorliegenden Erfindung gegen Schwankungen des pH bei der Verarbeitung, insbesondere bei der Entwicklung, vergleichsweise unempfindlich.

Die Kombination von ausgezeichneter Löslichkeit in organischen Lösungsmitteln und hervorragender Diffusionsfestigkeit einerseits mit guter Wasserverträglichkeit andererseits ist eine besonders überraschende, unvorhersehrbare Eigenschaft der erfindungsgemäßen Kuppler.

Die US-A-4 133 958 beschreibt eine Verbindung (Verbindung 50), ber der Y nicht eine p-alkoxysubstituierte, sondern eine o-alkoxysubstituierte Phenylgruppen bedeutet.

Bei der Herstellung des lichtempfindlichen farbfotografischen Aufzeichnungsmaterials können die diffusionsfesten Gelbkuppler der vorliegenden Erfindung in bekannter Weise in die Gießlösung der Silberhalogenidemulsionsschichten oder anderer Kolloidschichten eingearbeitet werden. Beispielsweise können die öllöslichen oder hydrophoben Gelbkuppler vorzugsweise aus einer Lösung in einem geeigneten Kupplerlösungsmittel (Ölbildner) gegebenenfalls in Anwesenheit eines Netz- oder dispergiermittels zu einer hydrophilen Kolloidlösung zugefügt werden. Die hydrophile Gießlösung kann selbstverständlich neben dem Bindemittel andere übliche Zusätze enthalten. Die Lösung des Farbkupplers braucht nicht direkt in die Gießlösung für die Silberhalogenidemulsionsschicht oder eine andere wasserdurchlässige Schicht dispergiert zu werden; sie kann vielmehr auch vorteilhaft zuerst in einer wäßrigen nicht-lichtempfindlichen Lösung eines hydrophilen Kolloids dispergiert oder gelöst werden, worauf das erhaltene Gemisch gegebenenfalls nach Entfernung der verwendeten organischen Lösungsmittel mit der Gießlösung für die lichtempfindliche Silberhalogenidemulsionsschicht oder einer anderen wasserdurchlässigen Schicht vor dem Auftragen vermischt wird.

Als lichtempfindliche Silberhalogenidemulsionen eignen sich Emulsionen von Silberchlorid, Silberbromid oder Gemische davon, evtl. mit einem geringen Gehalt an Silberiodid bis zu 10 mol-% in einem der üblicherweise verwendeten hydrophilen Bindemittel. Als Bindemittel für die fotografischen Schichten wird vorzugsweise Gelatine verwendet. Diese kann jedoch ganz oder teilweise durch andere natürliche oder synthetische Bindemittel ersetzt werden.

Die Emulsionen können in der üblichen Weise chemisch oder spektral sensibilisiert sein und die Emulsionsschichten wie auch andere nicht-lichtempfindliche Schichten können in der üblichen Weise mit bekannten Härtungsmitteln gehärtet sein.

Zur Herstellung farbfotografischer Bilder wird das erfindungsgemäße farbfotografische Aufzeichnungsmaterial, das in mindestens einer Silberhalogenidemulsionsschicht den neuen Gelbkuppler enthält, mit einer Farbentwicklerverbindung entwickelt. Als Farbentwicklerverbindung lassen sich sämtliche Entwicklerverbindungen verwenden, die die Fähigkeit besitzen in Form ihres Oxidationsproduktes mit Farbkupplern zu Azomethinfarbstoffen zu reagieren. Geeignete Farbentwicklerverbindungen sind aromatische mindestens eine primäre Aminogruppe enthaltende Verbindungen vom p-Phenylendiamintyp, beispielsweise N,N-Dialkyl-p-phenylendiamine, wie N,N-Di-ethyl-p-phenylendiamin, 1-(N-ethyl-N-methylsulfonamidoethyl)-3-methyl)-p-phenylendiamin, 1-(N-ethyl-N-hydroxyethyl)-3-methyl)-p-phenylendiamin und 1-(N-ethyl-N-methoxyethyl)-3-methyl)-p-phenylendiamin.

**Beispiel 1**

Farbfotografische Aufzeichnungsmaterialien 1 bis 6 wurden hergestellt, indem auf einen transparenten Schichtträger als Cellulosetriacetat die folgenden Schichten in der angegebenen Reihenfolge aufgetragen wurden. Die Mengenangaben beziehen sich jeweils auf 1 $m^2$. Für den Silberhalogenidauftrag werden die entsprechenden Mengen $AgNO_3$ angegeben.

Schicht 1 Silberbromidiodidemulsion (6 mol-% Iodid) mit 2,5 g $AgNO_3$, 2,5 g Gelatine und 2,5 mmol eines der in der folgenden Tabelle 1 aufgeführten Gelbkuppler; die Gelbkuppler wurden unter Verwendung der gleichen Gewichtsmenge Dibutylphthalat, d.h. als 1 : 1-Gemisch, in der Emulsion dispergiert.

Schicht 2 1,5 g Gelatine und 0,7 g Härtungsmittel der folgenden Formel

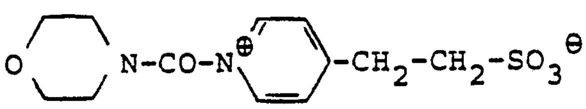 x $H_2O$

Je ein Probestreifen der Aufzeichnungsmaterialien 1 bis 6 wurde hinter einem Stufenkeil belichtet und bei 38°C einer Umkehrverarbeitung unterzogen wie beschrieben in The British Journal of Photography, 1981, Seiten 889 ff und unter Verwendung des folgenden Farbentwicklers (F 1)

| | |
|---|---|
| Nitrilotriessigsäure | 2 g |
| Trinatriumphosphat sicc. | 20 g |
| Kaliumbromid sicc. | 1 g |
| Kaliumiodid, 0,1 %-ige Lösung | 20 ml |
| Natriumsulfit sicc. | 4,5 g |
| 2,2'-Ethylendithiodiethanol | 1 g |
| 4-(N-Ethyl-N-methylsulfonamidoethyl)-2-methyl-phenylendiamin-Sesquisulfat-Monohydrat | 11 g |
| Zitrazinsäure | 1,3 g |
| auffüllen mit Wasser auf 1000 ml: pH | 11,6 |

Die erhaltenen Werte (maximale Farbdichte Dmax, spezifische Empfindlichkeit $E_{sp}$ und Körnigkeit $\sigma_D \cdot 10^{-2}$) sind der folgenden Tabelle 1 zu entnehmen. Die spezifische Empfindlichkeit $E_{sp}$ wird bestimmt bei der Dichte

$$D = \frac{1}{2}(Dmax + Dmin)$$

und wird in relativen log I.t-Werten angegeben.

Zur Messung der Körnigkeit wird auf T.H. James, The Theory of the Photographic Process, 4. Aufl. (1977), S. 618 - 621 verwiesen.

**Tabelle 1**

| Material | Kuppler | Dmax | $E_{sp}$ | $\sigma_D \cdot 10^{-2}$ |
|---|---|---|---|---|
| 1 | A | 2,45 | 22,5 | 4,3 |
| 2 | B | 2,68 | 21,1 | 5,7 |
| 3 | 4 | 2,91 | 21,7 | 4,6 |
| 4 | 5 | 3,15 | 21,3 | 4,1 |
| 5 | 16 | 3,38 | 21,3 | 5,0 |
| 6 | 31 | 3,35 | 21,3 | 4,8 |

Bei gleichem Silberauftrag liefern die erfindungsgemäßen Farbkuppler höhere maximale Farbdichten als die Vergleichskuppler A und B. Dabei ist die Farbkörnigkeit gegenüber dem Vergleichskuppler sogar verbessert.

**Vergleichskuppler A**

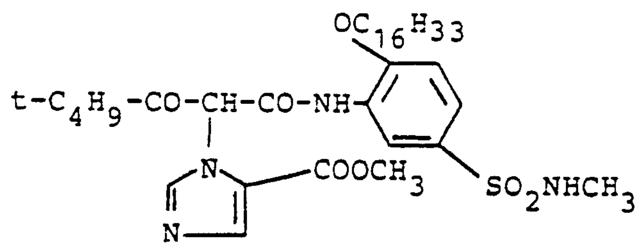

( = Kuppler 11 aus US-A-4 049 458)

**Vergleichskuppler B**

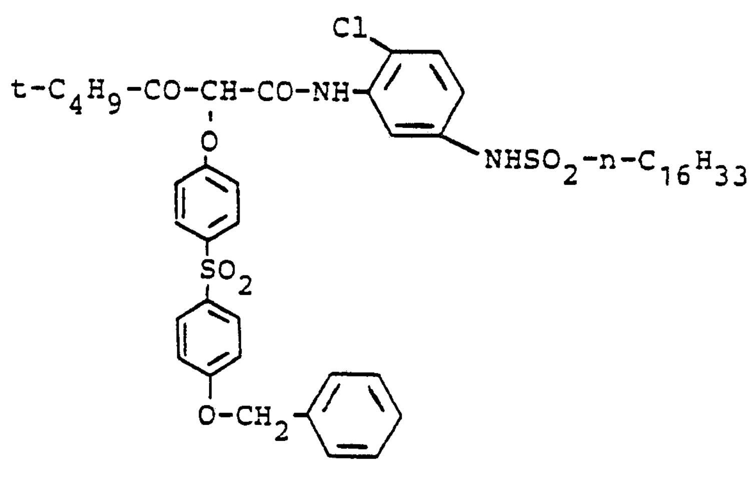

( = Kuppler 12 aus DE-A-2 456 076)

**Beispiel 2**

Je ein weiterer Probestreifen der in Beispiel 1 beschriebenen Aufzeichnungsmaterialien wurde hinter einem Stufenkeil belichtet und einer Negativentwicklungsverarbeitung unterzogen wie beschrieben in The British Journal of Photography, 1974, S. 597 ff. Die Ergebnisse sind der folgenden Tabelle 2 zu entnehmen.

**Tabelle 2**

| Material | Kuppler | Dmax | Dmin | Schleier-% | $E_{sp}$ |
|---|---|---|---|---|---|
| 1 | A | 3,15 | 0,76 | 24 | 28,3 |
| 2 | B | 3,25 | 0,62 | 19 | 25,9 |
| 3 | 4 | 4,23 | 0,60 | 14 | 26,9 |
| 4 | 5 | 4,43 | 0,58 | 13 | 26,9 |
| 5 | 16 | 4,28 | 0,43 | 10 | 28,6 |
| 6 | 31 | 4,67 | 0,33 | 7 | 28,2 |

Der Wert in der 5. Spalte gibt das Verhältnis Dmin/Dmax in Prozent an.

Die erfindungsgemäßen Gelbkuppler liefern nicht nur eine höhere maximale Farbdichte und damit eine höhere Farbausbeute, sondern auch einen geringen Schleier. Besonders deutlich drückt sich dies in dem Wert "Schleier-%" aus. Die höhere Farbausbeute bietet die Möglichkeit das Verhältnis $AgNO_3$/Kuppler durch Reduzierung des Silberauftrages zugunsten einer verbesserten Körnigkeit zu verändern. Gleichzeitig wird in den unter der blauempfindlichen gelbkupplerhaltigen Schicht angeordneten grün- bzw. rotempfindlichen Schichten die Schärfe verbessert.

**Beispiel 3**

Je ein Probestreifen der in Beispiel 1 beschriebenen Aufzeichnungsmaterialien 1 bis 6 wurde hinter einem Stufenkeil belichtet und wie in Beispiel 1 verarbeitet, wobei aber jeweils das verwendete Farbentwicklerbad mit KOH bzw. Phosphorsäure auf einen der pH-Werte 11,0; 11,3; 11,6 und 11,9 eingestellt wurde.
Die maximalen Farbdichten der entwickelten Probestreifen sind der folgenden Tabelle 3 zu entnehmen.

**Tabelle 3**

| Material | Kuppler | Dmax bei pH 11,0 | 11,3 | 11,6 | 11,9 |
|---|---|---|---|---|---|
| 1 | A | 2,43 | 2,45 | 2,45 | 2,35 |
| 2 | B | 2,02 | 2,40 | 2,68 | 2,93 |
| 3 | 4 | 2,81 | 2,93 | 2,91 | 2,88 |
| 4 | 5 | 3,00 | 3,06 | 3,15 | 3,07 |
| 5 | 16 | 3,26 | 3,37 | 3,38 | 3,25 |
| 6 | 31 | 3,32 | 3,34 | 3,35 | 3,17 |

Die maximalen Farbdichten der erfindungsgemäßen Gelbkuppler sind nicht nur höher als die der Vergleichskuppler A und B, sondern auch über einen relativ breiten pH-Bereich weitgehend konstant. Die erfindungsgemäßen Gelbkuppler sind damit in ihren fotografischen Abbildungseigenschäften weitgehend unabhängig von Schwankungen des pH-Wertes im Farbentwickler.

**Beispiel 4**

Wie in Beispiel 1 wurden farbfotografische Aufzeichnungsmaterialien 7 bis 11 hergestellt, jedoch mit folgender Schicht 1:

Schicht 1: Silberbromidiodidemulsion (3,8 mol-% Iodid) mit 1,0 g $AgNO_3$, 1,5 g Gelatine und 1,0 mmol eines der Gelbkuppler 5, 16, 31, A und B.

Von jedem der so hergestellten Aufzeichnungsmaterialien wurden zwei Probestreifen hinter einem Stufenkeil belichtet und wie in Beispiel 1 einer Umkehrverarbeitung, jedoch mit ausgetauschten Farbentwicklerbädern, unterzogen. Jeweils ein Streifen davon wurde in einem Farbentwickler (F 2) der folgenden Zusammensetzung entwickelt:

| | | |
|---|---|---|
| Nitrilotriessigsäure | 2 | g |
| Trinatriumphosphat sicc. | 20 | g |
| Kaliumbromid | 1 | g |
| Kaliumiodid, 0,1 %-ig | 20 | ml |
| Natriumsulfit | 4,5 | g |
| 3,6-Dithiooctandiol-1,8 | 1 | g |
| N-Ethyl-N-hydroxyethyl-3-methyl-p-phenylendiaminsulfat-monohydrat | 8 | g |
| auffüllen mit Wasser auf 1000 ml | | |

Der zweite Streifen wurde in einem Farbentwickler (F 3) entwickelt, der die gleiche Zusammensetzung wie der Farbentwickler (F 2) hat, jedoch als konkurrierenden Kuppler zusätzlich 1,3 g Zitrazinsäure enthielt. Beide Farbentwickler wurden mit KOH bzw. Phosphorsäure auf pH 11,6 eingestellt.

In der folgenden Tabelle 4 sind für jeden der untersuchten Gelbkuppler die bei Entwicklung in den beiden Farbentwicklern erhaltenen Dmax-Werte sowie der KRV-Wert angegeben, der nach folgender Gleichung ermittelt wurde:

$$KRV = \frac{Dmax\ (F\ 2)}{Dmax\ (F\ 3)}$$

KRV ist das "konkurrierte Reaktionsverhältnis", ausgedrückt als Quotient aus maximaler Farbdichte, erhalten in Abwesenheit eines konkurrierenden Kupplers, zu maximaler Farbdichte, erhalten in Gegenwart eines konkurrierenden Kupplers. Das Verfahren zur Ermittlung des KRV-Wertes ist in DE-A-2 456 076 beschrieben.

**Tabelle 4**

| Material | Kuppler | Dmax (F 2) | Dmax (F 3) | KRV |
|---|---|---|---|---|
| 7 | A | 1,47 | 1,37 | 1,07 |
| 8 | B | 1,38 | 1,31 | 1,05 |
| 9 | 5 | 1,62 | 1,61 | 1,01 |
| 10 | 16 | 1,46 | 1,45 | 1,00 |
| 11 | 31 | 1,45 | 1,44 | 1,00 |

Die erfindungsgemäßen Gelbkuppler erreichen nahezu den theoretischen Grenzwert 1,00 für den KRV-Wert.

**Beispiel 5**

Wie in Beispiel 1 wurden farbfotografische Aufzeichnungsmaterialien 12 bis 15 hergestellt, jedoch mit folgender Schicht 1:

Schicht 1 Silberbromidiodidemulsion (4,2 mol-% Iodid) gold- und schwefelgereift, die auf 40,8 g $AgNO_3$ 30,6 g Gelatine, 480 mg 4-Hydroxy-6-methyl-1,3,3a,7-tetraazainden, 20 mg eines Sensibilisators der folgenden Formel

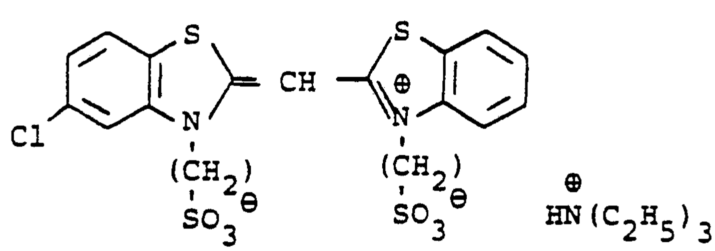

24 mg 3-Mercapto-5-(2-furyl)-1,2,4-triazol (Stabilisator gemäß DE-A 27 11 942) und 39,2 · $10^{-3}$ mol eines der vier in der folgenden Tabelle 5 angegebenen Gelbkuppler enthält.

Die vier verschiedenen Emulsionsproben wurden auf einen Schichtträger in einer solchen Menge aufgetragen, daß bei Verarbeitung gemäß The British Journal of Photographie, 1981, Seiten 889 ff vergleichbare maximale Farbdichten erhalten wurden. Die erforderlichen Silberaufträge (g $AgNO_3/m^2$) wurden durch Vorversuche ermittelt.

Je ein Probestreifen der verschiedenen Aufzeichnungsmaterialien wurde vor der Belichtung 14 d bei 57°C und 35 % relativer Feuchte gelagert. Zum Vergleich wurde ein weiterer Probestreifen von jedem der Aufzeichnungsmaterialien während der selben Zeitspanne bei +10°C gelagert. Anschließend wurden die Probestreifen hinter einem Stufenkeil belichtet und dem angegebenen Umkehrverarbeitungsverfahren

unterworfen. Der Silberauftrag und die maximale Farbdichte der klimagelagerten Probestreifen, angegeben in % bezogen auf die maximale Farbdichte der entsprechenden kühl gelagerten Probestreifen, ist in der folgenden Tabelle 5 aufgeführt.

**Tabelle 5**

| Material | Kuppler | g $AgNO_3/m^2$ | rel Dmax (%) |
|---|---|---|---|
| 12 | A | 3,3 | 45 |
| 13 | C | 3,6 | 73 |
| 14 | 4 | 2,3 | 80 |
| 15 | 5 | 2,1 | 87 |

Die erfindungsgemäßen Gelbkuppler sind den Vergleichskupplern deutlich überlegen, sowohl hinsichtlich einer verbesserten Lagerstabilität als auch hinsichtlich eines erheblich geringeren Bedarfes an Silberhalogenid zur Erzielung vergleichbarer maximaler Farbdichten.

**Vergleichskuppler C**

5 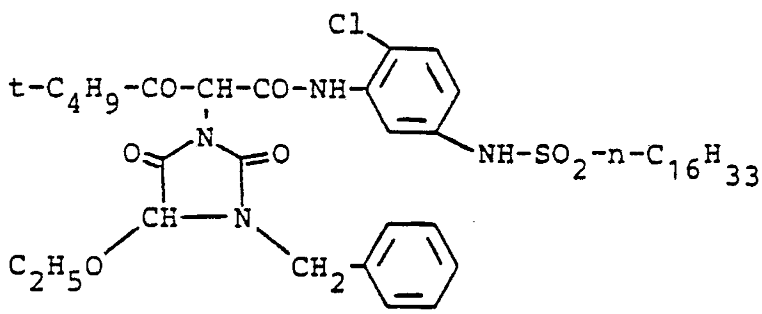

( = Kuppler 3 aus DE-A-2 713 022)

**Beispiel 6**

Farbfotografische Aufzeichnungsmaterialien 16 bis 19 wurden hergestellt wie in Beispiel 5, wobei jedoch der für Schicht 1 angegebenen Emulsion anstelle der dort erwähnten Kupplermenge einer der Gelbkuppler 4, 5, D und E in einer Menge von 5 · $10^{-2}$ mol zugefügt wurden. Der Vergleichskuppler E wurde in Form einer 5 %-igen wäßrig-alkalischen Lösung zugesetzt.

**Vergleichskuppler D**

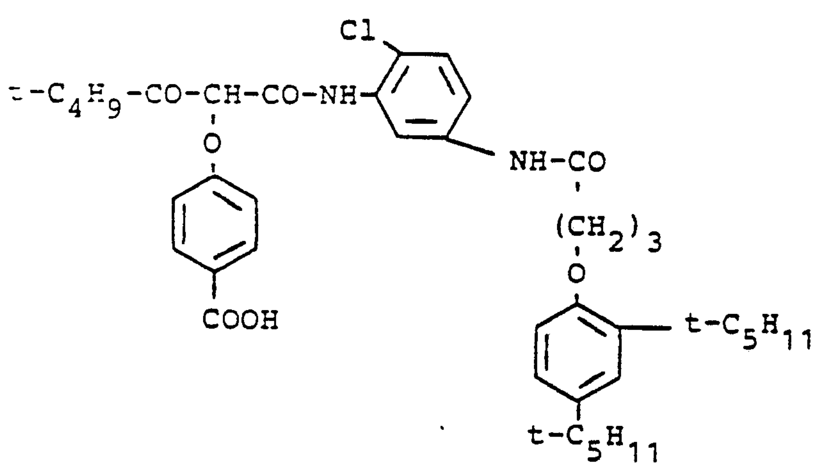

**Vergleichskuppler E**

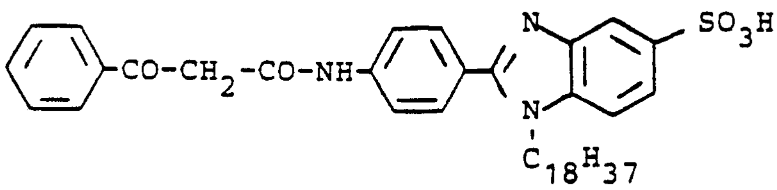

Im übrigen wird verfahren wie in Beispiel 5 beschrieben. Die angewendete Umkehrverarbeitung wird jedoch insofern abgeändert, als die Schlußwässerung um den Faktor 3 verlängert wird um den Gehalt an Restchemikalien aus den Verarbeitungsbädern möglichst gering zu halten und hierdurch verursachte Sekundäreffekte bei der anschließenden Farbstoff-Stabilitätsprüfung weitgehend zu vermeiden. Bei der anschließenden densitometrischen Ausmessung der Probestreifen wurde diejenige Stufe des aufbelichteten Keils markiert, die eine Dichte von nahezu 1 (= 100 %) aufweist. Durch anschließende Lagerung bei 60°C und 90 % relativer Feuchte über einen Zeitraum von 16 Wochen wurde eine "zeitgeraffte Stabilitätsprüfung" durchgeführt. Jeweils im Abstand von 2 Wochen wurde die Dichte der markierten Stufe gemessen und in % der ursprünglichen Dichte registriert. Das Ergebnis der "zeitgerafften Stabilitätsprüfung" ist in Fig. 1 dargestellt. Die Ordinate gibt die Dichte der markierten Stufe in % der ursprünglichen Dichte (D ≈ 1) wieder. Auf der Abszisse ist die Zeit in Wochen angegeben. Die aus den erfindungsgemäßen Gelbkupplern erhaltenen Farbstoffe sind deutlich stabiler als diejenigen, die aus den Vergleichskupplern erhalten worden sind.

**Patentanspruch**

Farbfotografisches Aufzeichnungsmaterial mit mindestens einer lichtempfindlichen Silberhalogenidemulsionsschicht und einem dieser zugeordneten nicht diffundierenden Gelbkuppler, dadurch gekennzeichnet, daß der Gelbkuppler der folgenden Formel entspricht

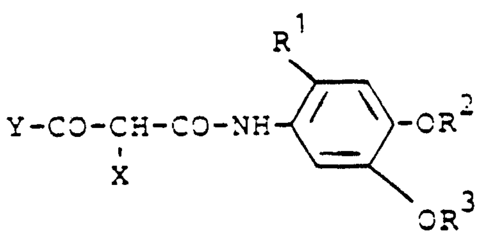

worin bedeuten:

Y eine p-Alkoxyphenylgruppe einer der folgenden Formeln:

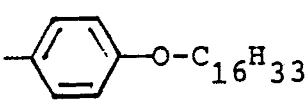

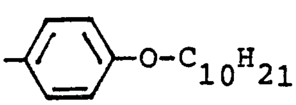

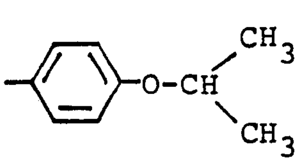

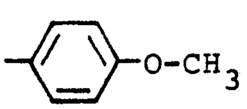

X ein Wasserstoffatom oder eine bei Farbkupplung abspaltbare Gruppe;
$R^1$ ein Halogenatom;
$R^2$ und $R^3$ gleiche oder verschiedene Alkylreste mit je 1 bis 20 C-Atomen, einschließlich substituierter Alkylreste.

**Claim**

Colour photographic recording material containing at least one light sensitive silver halide emulsion layer and a non-diffusible yellow coupler associated therewith, characterised in that the yellow coupler corresponds to the following formula:

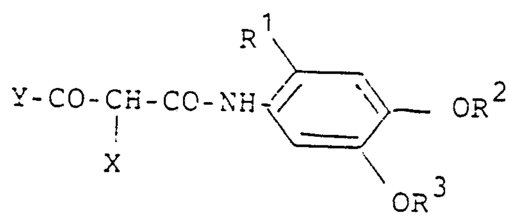

wherein

Y denotes a p-alkoxyphenyl group corresponding to one of the following formulae:

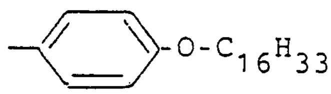

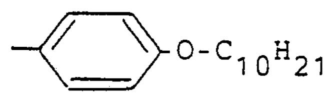

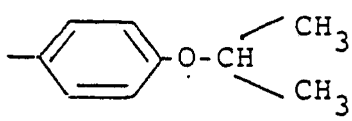

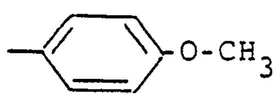

X denotes a hydrogen atom or a group capable of being split off in a coupling reaction;
$R^1$ denotes a halogen atom;
$R^2$ and $R^3$ denote identical or different alkyl groups each having 1 to 20 carbon atoms, including substituted alkyl groups.

**Revendication**

Support d'enregistrement pour la photographie en couleur, portant au moins une couche photosensible d'émulsion à l'halogénure d'argent en association avec un copulant jaune non diffusible, caractérisé en ce que le copulant jaune répond à la formule générale

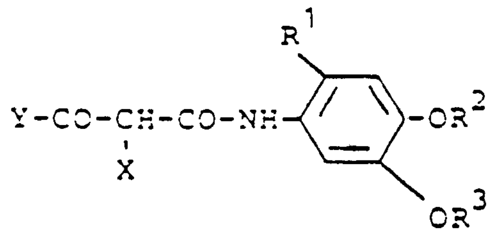

dans laquelle:

Y désigne un groupe p-alkoxyphényle répondant à l'une des formules suivantes:

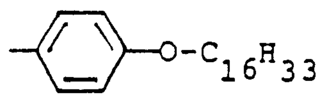

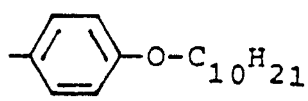

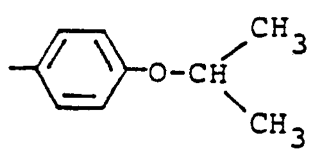

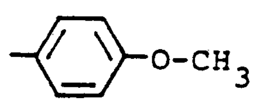

X est un atome d'hydrogène ou un groupe éliminable lors de la copulation chromogène;
$R^1$ est un atome d'halogène;
$R^2$ et $R^3$ sont des restes alkyle identiques ou différents ayant chacun 1 à 20 atomes de carbone, y compris des restes alkyle substitués.

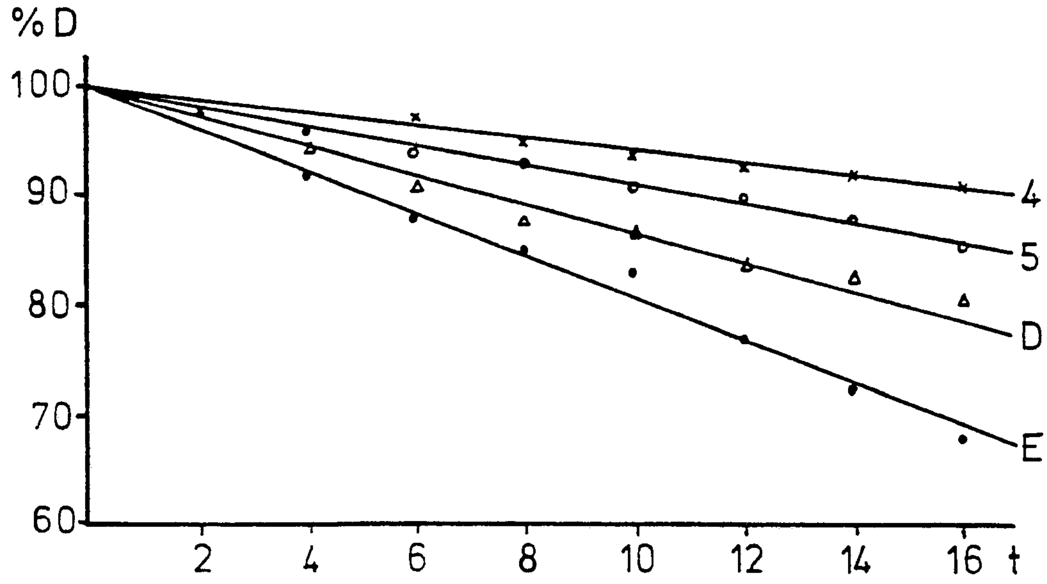


FIG. 1